(19) Europäisches Patentamt
European Patent Office
Office européen des brevets

(11) Veröffentlichungsnummer: **0 225 433 B1**

(12) EUROPÄISCHE PATENTSCHRIFT

(45) Veröffentlichungstag der Patentschrift: **28.07.93**

(51) Int. Cl.[5]: **C07D 251/70**, C08K 5/34

(21) Anmeldenummer: **86111945.1**

(22) Anmeldetag: **29.08.86**

Die Akte enthält technische Angaben, die nach dem Eingang der Anmeldung eingereicht wurden und die nicht in dieser Patentschrift enthalten sind.

# (54) Hydroxyoxaalkylmelamine, Verfahren zu ihrer Herstellung und deren Verwendung.

(30) Priorität: **07.09.85 DE 3531912**

(43) Veröffentlichungstag der Anmeldung:
**16.06.87 Patentblatt 87/25**

(45) Bekanntmachung des Hinweises auf die Patenterteilung:
**28.07.93 Patentblatt 93/30**

(84) Benannte Vertragsstaaten:
**CH DE FR GB LI**

(56) Entgegenhaltungen:
**EP-A- 0 051 734**
**US-A- 2 381 121**
**US-A- 4 312 988**

**A. WEISSBERGER: "Heterocyclic compounds with three- and four-membered rings", Auflage 1, 1964, Seite 289, Interscience Publishers, New York, US**

(73) Patentinhaber: **BASF Aktiengesellschaft**
**Carl-Bosch-Strasse 38**
**W-6700 Ludwigshafen(DE)**

(72) Erfinder: **Ebel, Klaus, Dr.**
**Ungsteiner Strasse 18**
**W-6700 Ludwigshafen(DE)**
Erfinder: **Reuther, Wolfgang, Dr.**
**Am Pferchelhang 16**
**W-6900 Heidelberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft neue Hydroxioxaalkylmelamine, insbesondere N-Mono-, N,N'-Bis- und N,N' ,N''-Tris-(5-hydroxi-3-oxapentyl)melamin, Verfahren zur Herstellung der reinen Verbindungen und von Gemischen dieser Verbindungen, sowie deren Verwendung.

Aus der US-A-4 312 988 ist die Umsetzung von Melamin mit Ethanolamin bzw. mit Isopropanolamin bekannt. Dort wurde gefunden, daß bei der Reaktion von Melamin mit Ethanolamin anstelle von N,N' ,N''-Tris-(2-hydroxiethyl)melamin in einer Konkurrenzreaktion unter Wasserabspaltung hauptsächlich Isomelamine gebildet werden, die für die geringe Ausbeute an N,N' ,N''-Tris-(2-hydroxiethyl)melamin verantwortlich sind. Beispielsweise wurden bei der Umsetzung von Ethanolamin mit Melamin im Molverhältnis 3,2:1 bei 82 % Umsatz 20 % an Isomelaminen, bei 95 % Umsatz bereits 50 % an Isomelaminen und bei 99 % Umsatz schließlich 100 % an Isomelaminen erhalten.

Es wurde daher vorgeschlagen, anstelle von Ethanolamin Isopropanolamin zu verwenden. Nach der Lehre der US-A-4 312 988 sollte nämlich die Isomelaminbildung drastisch reduziert werden können, wenn man das "gradkettige" Ethanolamin durch ein "verzweigtes" Isoalkanolamin ersetzt.

Aus dem dort beschriebenen Beispiel, in dem Isopropanolamin und Melamin umgesetzt werden, geht aber hervor, daß man dabei ein Endprodukt erhält, dessen Anteil an den unerwünnschten Isomelaminen immer noch 40 % beträgt.

Gegenstand der Erfindung sind Hydroxioxaalkylmelamine der allgemeinen Formel I

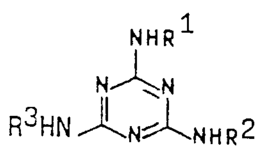

(I)

worin $R^1$ einen Rest der allgemeinen Formel II bedeutet:

$H\text{-}(\text{-}O\text{-}CHR'\text{-}CHR'\text{-})\text{-}_n$ (II) ,

in der die Reste R' Gleich oder verschieden sein können und für Wasserstoff oder $C_1$-$C_4$-Alkyl stehen und n die Bedeutungen 2 oder 3 besitzt und die Reste $R^2$ und $R^3$ für einen der Reste $R^1$ oder Wasserstoff stehen.

Bevorzugt sind Hydroxioxaalkylmelamine, worin R in der allgemeinen Formel I für Wasserstoff oder den 5-Hydroxi-3-oxapentyl-Rest der Formel III:

$Ho\text{-}CH_2\text{-}CH_2\text{-}O\text{-}CH_2\text{-}CH_2\text{-}$ (III)

steht.

Besonders bevorzugt sind die Verbindungen N-Mono-(5-hydroxi-3-oxapentyl)melamin, N,N'-Bis-(5-hydroxi-3-oxapentyl)melamin, N,N',N''-Tris-(5-hydroxi-3-oxapentyl)melamin, sowie deren Mischungen.

Die Verbindungen der allgemeinen Formel IV ergeben sich bevorzugt im Zuge der Aminierung von Ethylenoxid oder Propylenoxid. Daher stehen die Reste R' im Rest der allgemeinen Formel II für Wasserstoff oder Methyl, insbesondere für die Kombination R'/R' = H/H oder H/$CH_3$.

Gegenstand der Erfindung ist auch ein Verfahren zur Herstellung der Verbindung der allgemeinen Formel I, das dadurch gekennzeichnet ist, man das Melamin mit einer Verbindung der allgemeinen Formel IV:

$$H\text{-}(\text{-}O\text{-}CHR'\text{-}CHR'\text{-})_n\text{-}NH_2 \qquad (IV),$$

worin R' und n die zuvor genannten Bedeutungen besitzen, bei einer Temperatur von 120 bis 250 °C in Gegenwart eines sauren Katalysators umsetzt.

Überraschenderweise wurde gefunden, daß die nach dem bekannten Verfahren gebildeten Isomelamine beim Aminaustausch beispielsweise mit 2,2'-Aminoethoxiethanol vollständig ausgeschlossen werden können. Man erhält in quantitativer Ausbeute und über 95 %iger Reinheit N,N',N''-Tris-(5-hydroxy-2-oxapentyl)-melamin. Wenn die Umsetzung abgebrochen wird, kann man auch definierte Gemische aus der Tris- und der Bis-Verbindung oder aus der Tris-, Bis- und Mono-Verbindung reproduzierbar herstellen.

Gegenüber der Umsetzung mit den niedriger siedenden Alkanolaminen Ethanolamin (Kp. = 170 ° C) und Isopropanolamin (Kp. = 160 ° C) nach dem Stand der Technik bringt das höher siedende 2,2'-Aminoethoxiethanol zusätzlich noch den Vorteil kürzerer Reaktionszeiten (Kp. = 218 ° C).

Das Verfahren wird zweckmäßigerweise so durchgeführt, daß man ein Gemisch aus Melamin, 2,2'-Aminoethoxiethanol (Formel: $H_2N$-$CH_2$-$CH_2$-O-$CH_2$-$CH_2$-OH), saurem Katalysator und gegebenenfalls einem Lösungsmittel vorlegt und unter Rühren auf eine Temperatur von 120 bis 250 ° C, insbesondere von 150 bis 230 ° C erhitzt.

Man arbeitet im allgemeinen bei atmosphärischem Druck, um jedoch den oberen Temperaturbereich (230 bis 250 ° C) zu erreichen, muß ein Druck von 1 bis 15 bar eingehalten werden.

Weiterhin empfiehlt es sich, die Umsetzung in Gegenwart eines Schutzgases durchzuführen. Das Schutzgas wird dabei im allgemeinen über die Oberfläche der Reaktionsmischung geleitet. Geeignete Schutzgase sind z.B. Edelgase und insbesondere Stickstoff.

Als saure Katalysatoren kommen alle starken und mittelstarken Säuren in Betracht, z.B. Flußsäure, Salzsäure, Bromwasserstoffsäure, Schwefelsäure, Salpetersäure, Phosphorsäure, Amidosulfonsäure, Thiocyansäure, p-Toluolsulfonsäure oder Methansulfonsäure.

Die Säuren können entweder in freier Form oder als Melamin- oder Aminsalz zugegeben werden. Weiterhin ist auch die Zugabe als Salz einer Base, die schwächer als 2,2'-Aminoethoxiethanol ist, möglich, beispielsweise als Ammoniumsalz.

Anstelle der genannten Protonsäure katalysieren auch Lewis-Säuren, wie Bortrifluorid, Aluminiumchlorid, Zinn(IV)chlorid, Antimon(V)fluorid oder Eisen(III)bromid die Reaktion.

Pro Mol Melamin werden zweckmäßig 0,05 bis 3 Mol, vorzugsweise 0,1 bis 1 Mol Katalysator eingesetzt. Mit zunehmender Katalysatormenge ist ein Anstieg der Reaktionsgeschwindigkeit zu beobachten.

Das erfindungsgemäße Verfahren wird vorzugsweise in Abwesenheit von Lösungsmitteln durchgeführt, jedoch ist es auch möglich in einem organischen Lösungsmittel zu arbeiten. Hierfür sind Polyole, z.B. Ethylenglykol, 1,2-Propylenglykol, Diethylenglykol oder Triethylenglykol geeignet.

Die verwendete Menge an 2,2'-Aminoethoxiethanol kann beliebig gewählt werden. Vorzugsweise wird jedoch mit einem Aminüberschuß gearbeitet. Es werden gewöhnlich 3 bis 10 Mol Amin pro Mol Melamin eingesetzt.

Der Reaktionsverlauf kann mit analytischen Methoden, beispielsweise mittels Hochdruckflüssigkeitschromatographie (HPLC) verfolgt werden. Man kann die Reaktion bei jedem beliebigen Umsatzgrad abbrechen, wobei man Gemische aus N,N',N''-Tris-(5-hydroxi-3-oxapentyl)melamin, N,N'-Bis-(5-hydroxi-3-oxapentyl)melamin und gegebenenfalls auch N-Mono-(5-hydroxi-3-oxapentyl)melamin erhält, die eine definierte, reproduzierbare Zusammensetzung aufweisen.

Bei vollständigem Umsatz erhält man reines N,N',N''-Tris-(5-hydroxi-3-oxapentyl)melamin.

Zur Isolierung der Wertprodukte neutralisiert man zweckmäßig die jeweilige Katalysatorsäure, indem man eine Base, z.B. Natriumhydroxid, Kaliumhydroxid, Natriumcarbonat, Kaliumcarbonat, Calciumcarbonat oder Bariumcarbonat zur Reaktionsmischung gibt und die ausgefallenen Salze abtrennt.

Danach wird überschüssiges freies 2,2'-Aminoethoxiethanol bei vermindertem Druck (ca. 15 mbar) bei einer Temperatur von ca. 180 ° C abdestilliert, wobei der annähernd farblose Rückstand zu einem Harz erstarrt.

Nach einer weiteren Ausführungsform betrifft die Erfindung ein alternatives Verfahren zur Herstellung der Verbindungen gemäß der allgemeinen Formel I. Das Verfahren ist dadurch gekennzeichnet, daß man ein Chlortriazin der allgemeinen Formel V

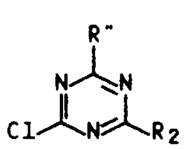

(V)

worin die Reste $R_2$ gleich oder verschieden sein können und für Cl oder $NH_2$ stehen,
in Gegenwart einer Base mit einer Verbindung der allgemeinen Formel IV:

$$H-(-O-CHR'-CHR'-)_n-NH_2 \qquad (IV),$$

worin R' und n die in Anspruch 1 angegebenen Bedeutungen besitzen, umsetzt.

Als Ausgangs-Chlortriazin verwendet man Diaminochlortriazin, Dichloraminotriazin oder Cyanurchlorid. Man erhält jeweils reine Verbindungen.

Als Base kann man eine beliebige Base einsetzen, zweckmäßig arbeitet man in Wasser unter Zugabe von Natronlauge (vgl. J.Am. Chem. Soc., 73, 2984-86 (1951)), oder in Dioxan unter Zugabe von Kaliumcarbonat (vgl. US-A-3 537 301).

Die Isolierung der Wertprodukte folgt auf übliche Weise.

Die als Ausgangsverbindungen eingesetzten Oxaalkanolamine der allgemeinen Formel II sind entweder bekannt oder lassen sich nach bekannten Methoden herstellen, vgl. M.S. Malinskii, A.N. Korchagina A.G. Yudasina, D.G. Yurko Vorpr. Khim. Khim. Teknol. 1974, 34, 6-11 (Russ.) und Jpn. Kokai JP-A-79 03 005 Anm. Nr. 77/67,803.

Die Chlortriazine der allgemeinen Formel V sind bekannte Verbindungen.

Die erfindungsgemäßen neuen Verbindungen sind wertvolle Zwischenprodukte zur Herstellung von Urethanen und eignen sich außerdem hervorragend zur Modifizierung von Aminoplastharzen.

Die nachfolgenden Beispiele sollen die Erfindung näher erläutern.

## Beispiel 1

### N,N',N''-Tris-(5-hydroxi-3-oxapentyl)melamin

Man kochte 111,0 (0,6 Mol) Cyanurchlorid, 160 g wasserfreies Kaliumcarbonat und 210,0 g (2,0 Mol) 2,2'-Aminoethoxyethanol in 1,2 l Dioxan 5 h unter Rückfluß. Dann wurde heiß abgesaugt und das Filtrat zur Trocknung eingedampft. Man erhielt 229 g (98 %) eines schwach gelben Harzes. Zur Analyse wurde aus Wasser kristallisiert. Man erhielt farblose Kristalle vom Schmp. 68 °C.

$^1$H-NMR

S (ppm) = 3,4 ppm (S, 24 H); 4,4 (S, br., 3 H)
6,4 ppm (S, br., 3 H)

Analyse

| | C | H | N | O |
|---|---|---|---|---|
| ber.: | 46,1 | 7,7 | 21,5 | 24,6 |
| gef.: | 45,8 | 7,6 | 21,2 | 24,9 |

## Beispiel 2

### N,N'-Bis-(5-hydroxi-3-oxapentyl)melamin

Man tropfte 47,3 g (0,45 Mol) 2,2'-Aminoethoxiethanol zur Aufschlämmung von 33,0 g (0,2 Mol) Dichloraminotriazin in 200 ml Wasser. Dann wurde auf 100 °C erhitzt und der pH-Wert durch Zugabe von 25 %iger Natronlauge zwischen 8 und 9 gehalten. Als keine Lauge mehr verbraucht wurde, ließ man abkühlen und dampfte im Vakuum zur Trocknung ein. Der Rückstand wurde mit 250 ml Isopropanol aufgekocht und das unlösliche Natriumchlorid abfiltriert. Das Filtrat wurde im Vakuum vom Lösungsmittel befreit und man erhielt 59 g (98 %) eines farblosen Harzes. Zur Analyse wurde am Kugelrohr destilliert (200 °C/0,1 mbar).

$^1$H-NMR

S (ppm) = 3,4 ppm (S, 16 H); 4,5 (S, br., 3 H); 6,0 (S, br., 2 H)
6,4 ppm (S, br., 2 H)

**Analyse**

| | C | H | N | O |
|---|---|---|---|---|
| ber.: | 43,7 | 7,3 | 27,8 | 21,2 |
| gef.: | 43,7 | 7,3 | 27,4 | 21,5 |

Beispiel 3

N-Mono-(5-hydroxi-3-oxapentyl)melamin

Man erhitzte 58,2 g (0,40 Mol) Diaminochlortriazin und 47,3 g (0,45 Mol) 2,2'-Aminoethoxiethanol in 300 ml Wasser unter Rückfluß und hielt den pH-Wert durch zudosieren von 25 %iger Natronlauge zwischen 8 und 9. Nach dem Ende der Reaktion ließ man die klare Lösung abkühlen, wobei das Produkt auskristallisierte. Nach dem Absaugen und Trocknen erhielt man 74,3 g (87 %) farblose Kristalle vom Schmp. 108 °C. Zur Analyse wurde aus Wasser umkristallisiert.

$^1$H-NMR

S (ppm) = 3,4 ppm (S, 8 H); 4,5 (S, br., 1 H); 6,0 (S, br., 4 H)
6,4 ppm (S, br., 1 H)

**Analyse**

| | C | H | N | O |
|---|---|---|---|---|
| ber.: | 39,2 | 6,6 | 39,2 | 14,9 |
| gef.: | 39,1 | 6,5 | 38,7 | 16,0 |

Beispiel 4

Herstellung von N,N',N''-Tris-(5-hydroxi-3-oxapentyl)melamin aus Melamin

Zur Mischung von 229 g (1,8 Mol) Melamin und 1530 g (15,6 Mol) 2,2'-Aminoethoxiethanol tropfte man unter Rühren 37,5 g (0,4 Mol) konz. Schwefelsäure. Dann wurde unter Rühren und Überleiten eines schwachen Stickstoffstromes bis zum vollständigen Umsatz am Rückfluß gekocht (210 bis 220 °C, etwa 10 bis 12 h). Schließlich wurde das Hydrosulfat durch Zugabe von 58,3 g 50 %iger Natronlauge zersetzt und das ausgefallene Natriumsulfat heiß abfiltriert. Durch Abdestillieren des Aminoethoxiethanol-Überschusses im Vakuum erhielt man 702 g (100 %) eines schwach gelben Harzes, das nach quantitativer HPLC-Analyse (Vergleichssubstanz siehe Beispiel 1) zu 96 bis 98 % aus reinem N,N',N''-Tris-(5-hydroxi-3-oxapentyl)-melamin bestand. Auch das $^1$H-NMR-Spektrum stimmte mit dem Spektrum der Substanz aus Beispiel 1 überein.

Beispiel 5

Gemisch aus Mono-, N,N'-Bis- und N,N',N''-Tris-(5-hydroxi-3-oxapentyl)-melamin

Zur Mischung von 504 g (4,0 Mol) Melamin, 1682 g (16,0 Mol) 2,2'-Aminoethoxiethanolund 993 g (16,0 Mol) Ethylenglykol tropfte man unter Rühren 78,4 g (0,8 Mol) konz. Schwefelsäure. Dann wurde unter Rühren und Überleiten eines schwachen Stickstoffstromes etwa 3 h unter Rückfluß gekocht (200 bis 210 °C). Zur Aufarbeitung wurden 128 g (0,8 Mol) 50 %ige Natronlauge zugegeben und das ausgefallene Natriumsulfat abfiltriert. Schließlich wurden das überschüssige Amin und das Glykol im Vakuum abdestilliert und man erhielt 1150 g (100 %) eines schwach gelben Harzes mit der nachfolgenden, durch HPLC bestimmten Zusammensetzung:
etwa
4 Mol % Melamin,

26 Mol % Mono-(5-hydroxi-3-oxapentyl)melamin,
52 Mol % N,N'-Bis-(5-hydroxi-3-oxapentyl)melamin und
18 Mol % N,N',N''-Tris-(5-hydroxi-3-oxapentyl)melamin.

Beispiel 6

Gemisch aus N,N'-Bis- und N,N',N''-Tris-(5-hydroxi-3-oxapentyl)melamin

Zur Mischung von 252 g (2,0 Mol) Melamin, 1050 g (10,0 Mol) 2,2'-Aminoethoxiethanolund 372 g (6,0 Mol) Ethylenglykol tropfte man unter Rühren 39,2 g (0,4 Mol) konz. Schwefelsäure. Nach analoger Durchführung der Reaktion und Aufarbeitung wie in Beispiel 5 mit 64 g (0,8 Mol) 50 %iger Natronlauge erhielt man nach etwa 6 h Reaktionszeit 727 g (100 %) eines Gemisches aus 40 % N,N'-Bis- und 60 % N,N',N''-Tris-(5-hydroxy-3-oxapentyl)melamin (HPLC).

**Patentansprüche**

**1.** Hydroxioxaalkylmelamine der allgemeinen Formel I

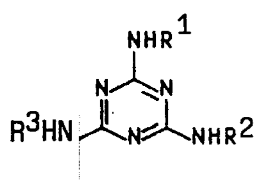

(I)

worin $R^1$ einen Rest der allgemeinen Formel II bedeutet:

$H-(-O-CHR'-CHR'-)_n$ (II) ,

in der die Reste R' gleich oder verschieden sein können und für Wasserstoff oder $C_1$-$C_4$-Alkyl stehen und n die Bedeutungen 2 oder 3 besitzt und die Reste $R^2$ und $R^3$ für einen der Reste $R^1$ oder Wasserstoff stehen.

**2.** Hydroxioxaalkylmelamine nach Anspruch 1, worin $R^1$ den 5-Hydroxi-3-oxapentyl-Rest der Formel III bedeutet:

$Ho-CH_2-CH_2-O-CH_2-CH_2-$ (III) .

**3.** Hydroxioxaalkylmelamine nach Anspruch 2, ausgewählt unter N-Mono-(5-hydroxi-3-oxapentyl)melamin, N,N'-Bis-(5-hydroxi-3-oxapentyl)melamin und N,N' ,N''-Tris-(5-hydroxi-3-oxapentyl)melamin.

**4.** Verfahren zur Herstellung der Verbindungen nach einem der Ansprüche 1 bis 3, unter Verwendung von Melamin, dadurch gekennzeichnet, daß man das Melamin mit einer Verbindung der allgemeinen Formel IV:

$$H-(-O-CHR'-CHR'-)_n-NH_2 \quad (IV),$$

worin R' und n die in Anspruch 1 angegebenen Bedeutungen besitzen, bei einer Temperatur von 120 bis 250 °C in Gegenwart eines sauren Katalysators umsetzt.

**5.** Verfahren nach Anspruch 4 , dadurch gekennzeichnet, daß man 0.05 bis 3 Mol Katalysator pro Mol Melamin einsetzt.

6. Verfahren zur Herstellung der reinen Verbindungen nach einem der Ansprüche 1 bis 3, dadurch gekennzeichnet, daß man ein Chlortriazin der allgemeinen Formel V

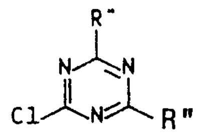

(V)

worin die Reste R" gleich oder verschieden sein können und für Cl oder $NH_2$ stehehn, in Gegenwart., einer Base mit einer Verbindung der allgemeinen Formel IV:

$$H-(-O-CHR'-CHR'-)_n-NH_2 \quad (IV),$$

worin R' und n die in Anspruch 1 angegebenen Bedeutungen besitzen, umsetzt.

7. Verwendung der Verbindungen gemäß einem der Ansprüche 1 bis 3, einzeln oder in Mischung, bei der Herstellung von Urethanen.

8. Verwendung der Verbindungen gemäß einem der Ansprüche 1 bis 3, einzeln oder in Mischung, zur Modifizierung von Aminoplastharzen.

## Claims

1. A hydroxyoxaalkylmelamine of the formula I

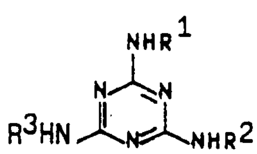

(I)

where $R^1$ is a radical of the formula II

$H-(-O-CHR'-CHR'-)-_n$ (II)

where the radicals $R^1$ may be identical or different and are each hydrogen or $C_1$-$C_4$-alkyl and n is 2 or 3, and the radicals $R^2$ and $R^3$ are one of the radicals $R^1$ or hydrogen.

2. A hydroxyoxaalxylmelamine as claimed in claim 1, wherein $R^1$ is the 5-hydroxy-3-oxapentyl radical of the formula III

$Ho-CH_2-CH_2-O-CH_2-CH_2-$ (III) .

3. A hydroxyoxaalkylmelamine as claimed in claim 2, selected from the group consisting of N-mono-(5-hydroxy-3-oxapentyl)-melamine, N,N'-bis-(5-hydroxy-3-oxapentyl)melamine and N,N',N"-tris-(5-hydroxy-3-oxapentyl)melamine.

4. A process for the preparation of a compound as claimed in any of claims 1 to 3, using melamine, wherein the melamine is reacted with a compound of the formula IV

$$H-(-O-CHR'-CHR'-)-_nNH_2 \qquad (IV)$$

where R' and n have the meanings stated in claim 1, at from 120 to 250 °C in the presence of an acidic catalyst.

**5.** A process as claimed in claim 4, wherein from 0.05 to 3 moles of catalyst are employed per mole of melamine.

**6.** A process for the preparation of a pure compound as claimed in any of claims 1 to 3, wherein a chlorotriazine of the formula V

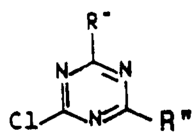

(V)

where the radicals R" may be identical or different and are each Cl or $NH_2$, is reacted with a compound of the formula IV

$$H-(-O-CHR'-CHR'-)-_nHN_2 \qquad (IV)$$

where $R^1$ and n have the meanings stated in claim 1, in the presence of a base.

**7.** The use of the compounds as claimed in any of claims 1 to 3, individually or as a mixture, for the preparation of urethanes.

**8.** The use of the compounds as claimed in any of claims 1 to 3, individually or as a mixture, for the modification of aminoplast resins.

**Revendications**

**1.** Hydroxyoxaalkylmélamines de formule générale I

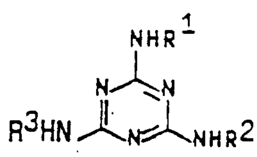

(I)

dans laquelle $R^1$ représente un reste de formule générale II

$H-(-O-CHR'-CHR'-)-_n$ (II)

où les restes R' peuvent être identiques ou différents et sont mis pour des atomes d'hydrogène ou des restes alkyle en $C_1$-$C_4$ et n représente le nombre 2 ou 3, et dans laquelle les restes $R^2$ et $R^3$ sont mis chacun pour l'un des restes $R^1$ ou pour un atome d'hydrogène.

**2.** Hydroxyoxaalkylmélamines selon la revendication 1, dans lesquelles $R^1$ représente le reste 5-hydroxy-3-oxapentyle de formule III

$HO-CH_2-CH_2-O-CH_2-CH_2-$ (III)

**3.** Hydroxyoxaalkylmélamines selon la revendication 2, choisies parmi la N-mono-(5-hydroxy-3-oxapentyl)-mélamine, la N,N'-bis(5-hydroxy-3-oxapentyl)mélamine et la N,N',N"-tris(5-hydroxy-3-oxapentyl)-mélamine.

**4.** Procédé de préparation des composés selon l'une quelconque des revendications 1 à 3, avec utilisation de mélamine, caractérisé en ce qu'on fait réagir la mélamine avec un composé de formule générale IV

$$H-(-O-CHR'-CHR'-)_n-NH_2 \qquad (IV)$$

dans laquelle R' et n ont les significations indiquées dans la revendication 1, à une température de 120 à 250 ° C en présence d'un catalyseur acide.

**5.** Procédé selon la revendication 4, caractérisé en ce qu'on utilise de 0,05 à 3 moles de catalyseur par mole de mélamine.

**6.** Procédé de préparation des composés purs selon l'une quelconque des revendications 1 à 3, caractérisé en ce qu'on fait réagir une chlorotriazine de formule générale V

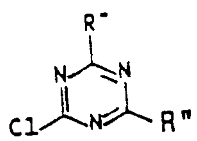

(V)

dans laquelle les restes R" peuvent être identiques ou différents et sont mis pour Cl ou $NH_2$, en présence d'une base, avec un composé de formule générale IV

$$H-(-O-CHR'-CHR'-)_n-NH_2 \qquad (IV)$$

dans laquelle R' et n ont les significations indiquées dans la revendication 1.

**7.** Utilisation des composés selon l'une quelconque des revendications 1 à 3, individuellement ou en mélange, dans la préparation d'urétnanes.

**8.** Utilisation des composés selon l'une quelconque des revendications 1 à 3, individuellement ou en mélange, pour la modification de résines aminoplastes.